# EUROPÄISCHE PATENTANMELDUNG

(11) **EP 4 141 125 A1**
(43) Veröffentlichungstag der Anmeldung: **01.03.2023**
(21) Anmeldenummer: 22191671.1
(22) Anmeldetag: 23.08.2022
(51) Int. Cl.: C12Q 1/46

(54) **TESTKIT ZUR DETEKTION VON ACETYLCHOLINESTERASEHEMMERN**

(30) Priorität: 24.08.2021 DE 202021104545 U
(71) Anmelder: Dr. Franz Köhler Chemie GmbH, 64625 Bensheim (DE)
(72) Erfinder: Dr. Köhler, Gernot, 64665 Alsbach-Hähnlein (DE); Weiß, Frauke, 64342 Seeheim-Jugenheim (DE)
(74) Vertreter: Blumbach · Zinngrebe Patentanwälte PartG mbB

(57) **Zusammenfassung**

Die Erfindung betrifft ein Testkit zur Detektion von Organophosphatverbindungen umfassend eine Trägervorrichtung (2), eine Probenentnahmevorrichtung (6), eine Pufferlösung (7) in einem Probenröhrchen (32), sowie einer Acetylcholinesterase als erste aktive Komponente und einer Mischung aus 5,5'-Dithiobis-2-nitrobenzoesäure (DTNB) und Acetylthiocholin, wobei die Probenentnahmevorrichtung (6) in einem Probenröhrchen (30) verpackt ist, die erste aktive Komponente in einem ersten Reagenziendeckel (42) und die zweite aktive Komponente in einem zweiten Reagenziendeckel (43) appliziert ist und der erste und der zweite Reagenziendeckel (42, 43) jeweils ein Probenröhrchen (33, 34) verschließt, wobei die Trägervorrichtung zumindest vier Fixierungsvorrichtungen (20, 21, 22, 23) zur Aufnahme der Probenröhrchen (30, 32, 33, 34) aufweist, wobei die Probenröhrchen parallel zur Oberseite der Trägervorrichtung (2) fixiert werden und wobei die Trägervorrichtung zumindest zwei Aufnahmevorrichtungen (201, 202) zur Aufnahme des ersten und des zweiten Reagenziendeckels (42, 43) und eine Aufnahmevorrichtung (203) zur vertikalen Aufnahme eines Probenröhrchens (30) aufweist.

## Beschreibung

### Gebiet der Erfindung

Die Erfindung betrifft ein Testkit zur Detektion von Acetylcholinesterasehemmern. Insbesondere betrifft die Erfindung ein mobiles Testkit zum Nachweis von Acetylcholinesterasehemmern durch eine Farbreaktion und visueller Detektion.

Bekannte Vertreter von Acetylcholinesterasehemmern sind beispielsweise die Phosphorsäureester, Tabun, Soman, Sarin, VX, Cyclosarin oder Novichok. Sie wirken auf den menschlichen Organismus, indem sie an das Enzym Acetylcholinesterase (AChE) binden und somit dessen Funktion stören. Als Folge davon kann der Neurotransmitter Acetylcholin nicht mehr abgebaut werden, so dass es zu einem Überfluss von Acetylcholin im synaptischen Spalt kommt. Dies führt zu einer Überstimulation des cholinergen Systems und somit zu einer Vielzahl körperlicher Symptome wie Pupillenverengung, Speichelfluss, Bradykardie, Krämpfen und Atemdepression. Abhängig von der Schwere einer Vergiftung kann dies zum Tod durch Ersticken führen.

Ein schneller Nachweis einer entsprechenden Vergiftung ist hierbei sowohl für das Einleiten von Gegenmaßnahmen beim Vergiftungsopfer als auch zur Detektion von Kontaminationen zur Vermeidung entsprechender Vergiftungen von entscheidender Bedeutung. Daher besteht ein Bedarf an mobilen Testkits, welche einen schnellen und zuverlässigen Nachweis am Einsatzort ermöglichen.

Aus dem Stand der Technik ist eine Nachweisreaktion bekannt, bei dem der Nachweis indirekt und visuell über eine Farbreaktion erfolgt. Hierbei wird zunächst eine Probe genommen und in einer Pufferlösung mit menschlicher Acetylcholinesterase, im Folgenden auch als hAChE bezeichnet, versetzt. Anschließend wird eine Mischung aus Acetylthiocholin und 5,5'-Dithiobis-2-nitrobenzoesäure (DTNB), auch als Ellmans Reagenz bezeichnet, zur Reaktionslösung gegeben. In Anwesenheit einer aktiven Acetylcholinesterase erfolgt eine Umsetzung des Acetylthiocholins zu Thiocholin, welches wiederum mit 5,5'-Dithiobis-2-nitrobenzoesäure unter Freisetzung von 2-Nitro-5-thiobenzoat (TNB-) reagiert. In basischen und neutralen Lösungen deprotoniert 2-Nitro-5-thiobenzoat (TNB-) zum gelben Farbstoff TNB²⁻. Dieses weist eine charakteristische, gelbe Färbung auf, so dass die Testlösung in Abhängigkeit von der Konzentration des freigesetzten 2-Nitro-5-thiobenzoat (TNB-) eine unterschiedlich stark ausgeprägte, gelbe Farbe aufweist. Erfolgt dagegen keine Aufspaltung des DTNB, weil die zugegebene Acetylcholinesterase gehemmt und somit kein Thiocholin gebildet wird, so bleibt die Lösung farblos. Anhand einer Farbtafel kann der Anwender somit ermitteln, ob in der genommenen Probe ein Acetylcholinesterasehemmer vorhanden ist.

Um eine schnelle Detektion und gegebenenfalls Dekontamination zu gewährleisten, ist ein schneller Nachweis von Acetylcholinesterasehemmern, der vor Ort erfolgen kann, notwendig. Aus dem Stand der Technik sind mobile Test-Kits bekannt, mit denen Acetylcholinesterasehemmer nach dem oben beschriebenen Prinzip vor Ort nachgewiesen werden können. Hierbei handelt es sich um ein Kit mit mehreren Einzelkomponenten, die üblicherweise zum Schutz vor Umwelteinflüssen in einem Folienbeutel verschweißt sind. Das Kit enthält als Einzelkomponenten ein Wattestäbchen zur Probenentnahme, welches zusätzlich in einem versiegelten Beutel aus Aluminiumverbundfolie eingeschweißt ist.

Weiterhin enthält das Testkit ein Probenröhrchen mit einer Pufferlösung, einen Deckel, in den Acetylcholinesterase appliziert wurde sowie einen Deckel, der die Substanzen Acetylthiocholin und DTNB enthält. Die beiden Deckel sind hierbei ebenfalls zusätzlich in einem versiegelten Folienbeutel verpackt. Nachteilig an den Testkits ist jedoch, dass diese auf Grund der Vielzahl an Einzelkomponenten unübersichtlich sind und zudem das Öffnen der einzelnen, verschweißten Folienbeutel zur Entnahme der einzelnen Komponenten zeitaufwendig ist und zudem eine gewisse motorische Fertigkeit voraussetzt, welche unter Umständen nicht gegeben ist, insbesondere, wenn der Anwender eine entsprechende Schutzausrüstung trägt. Weiterhin ist die Durchführung des Nachweises auf Grund der ungeordnet in der Umverpackung vorliegenden Einzelkomponenten fehleranfällig. Ein weiterer Nachteil des bekannten Testkits besteht bei der Verwendung von Aluminiumverbundfolien, da diese nicht oder nur bedingt autoklavierbar sind. So kann das Beutelmaterial beim Autoklavieren verkleben, so dass sich die einzelnen Komponenten gegebenenfalls nicht oder nur sehr schwer entnehmen lassen. Um dieses Problem zu umgehen, werden in anderen Testkits für die Autoklavierung geeignete Kunststoffbeutel verwendet. Da diese jedoch eine höhere Wasserdampfdurchlässigkeit aufweisen als die Aluminiumfolien, wird die Haltbarkeit des Testkits verringert.

### Aufgabe der Erfindung

Es ist Aufgabe der Erfindung, ein mobiles Testkit zum Nachweis von Acetylcholinesterasehemmern bereit zu stellen, welches die aus dem Stand der Technik bekannten Nachteile nicht aufweist. Insbesondere ist es Aufgabe der Erfindung, ein entsprechendes mobiles Testkit bereitzustellen, welches gut handhabbar ist und gleichzeitig eine lange Haltbarkeit aufweist. Diese Aufgabe wird überraschenderweise bereits durch den Gegenstand des unabhängigen Anspruchs gelöst. Vorteilhafte Ausgestaltungen und Weiterentwicklungen sind Gegenstand der Unteransprüche.

### Kurzbeschreibung der Erfindung

Das Testkit zur Detektion von Acetylcholinesteraseinhibitorn umfasst eine Trägervorrichtung, eine Probenentnahmevorrichtung, eine Pufferlösung in einem Probenröhrchen mit Deckel sowie als eine erste reaktive Komponente eine Acetylcholinesterase sowie als eine zweite reaktive Komponente eine Mischung aus Acetylthiocholin und DNTB.

Erste und zweite reaktive Komponente liegen jeweils in fester Form vor und sind jeweils in einem Reagenziendeckel appliziert, der zum Verschließen des Probenröhrchens mit der Pufferlösung geeignet ist. Die Reagenziendeckel mit der ersten und der zweiten reaktiven Komponente verschließen jeweils ein Probenröhrchen. Die Probenröhrchen dienen hierbei zum Schutz der in den Reagenziendeckeln applizierten, reaktiven Komponenten. So wird durch die Probenröhrchen sowohl ein Herausfallen der Substanzen, als auch eine Kontamination der reaktiven Komponenten verhindert. Gegenüber einer Folienverpackung bietet die Aufbewahrung der Reagenziendeckel aufgeschraubt auf einem Probenröhrchen mehrere Vorteile. So ist ein Abschrauben der Deckel vom Probenröhrchen einfacher als das Öffnen einer verschweißten Folienverpackung und das Herausholen der Reagenziendeckel aus der geöffneten Folienverpackung, so dass das erfindungsgemäße Testkit somit leichter handhabbar ist. Dies gilt insbesondere bei der Verwendung von Schutzhandschuhen, da hierdurch die Feinmotorik des Anwenders eingeschränkt wird.

Gemäß einer Ausführungsform der Erfindung enthält das Probenröhrchen mit dem Reagenziendeckel der ersten reaktiven Komponente und/oder das Probenröhrchen mit dem Reagenziendeckel der zweiten reaktiven Komponente ein Trockenmittel, bevorzugt Silika. Das Trockenmittel ist hierbei vorzugsweise umverpackt, beispielsweise in einem Beutel. Durch das Trockenmittel können die Reagenzien wirksam vor Umwelteinflüssen wie einer hohen Luftfeuchtigkeit geschützt werden, was sich vorteilhaft auf die Verwendungsdauer der Testkits auswirkt.

Auch die Probenentnahmevorrichtung, beispielsweise ein Wattestäbchen, ist beim erfindungsgemäßen Testkit nicht in einer Folienverpackung eingeschweißt, sondern in einem Probenröhrchen mit Deckel verpackt. Wie bereits oben beschrieben, führt dies zu einer gegenüber einer Folienverpackung deutlich vereinfachten Öffnung der Umverpackung der Probenentnahmevorrichtung. Die Probenentnahmevorrichtung kann hierbei durch ein leichtes Schütteln aus dem Probenröhrchen entfernt werden. Zudem ist ein Probenröhrchen deutlich stabiler als eine Folienverpackung, so dass beispielsweise eine Beschädigung der Umverpackung durch eine Kante der Probenentnahmevorrichtung vermieden wird. Zudem lässt sich die Probenentnahmevorrichtung bei der Fertigung leichter in ein Röhrchen einsetzen als in einer Folie einschweißen, so dass die Verwendung eines Probenröhrchens als Umverpackung auch in Hinblick auf die Herstellung des Testkits vorteilhaft ist.

Ein weiterer Vorteil der Verwendung von Proberöhrchen als Umverpackung der Einzelkomponenten liegt darin, dass im Gegensatz zu Folienbeuteln kein Verschweißen notwendig ist. Somit wird die Herstellung des Testkits vereinfacht. Zudem sind die verwendeten Probenröhrchen autoklavierbar und weisen gleichzeitig eine geringe Wasserdampfdurchlässigkeit auf, was sich vorteilhaft auf die Haltbarkeit bzw. den zulässigen Verwendungszeitraum des Testkits auswirkt. Zudem lassen sich Probenröhrchen zum Autoklavieren leichter positionieren als Folienbeutel, was den Autoklaviervorgang ebenfalls vereinfacht. Gemäß bevorzugten Ausführungsformen bestehen die Probenröhrchen z.B. aus einem Kunststoff wie Polypropylen, Polyethylen, Polystyrol oder Polycarbonat.

Gemäß einer vorteilhaften Ausgestaltung der Erfindung weisen die Probenröhrchen ein Schraubgewinde auf und alle Deckel sind als Schraubdeckel ausgebildet. Dies ermöglicht ein besonders einfaches, kontrolliertes Öffnen und Verschließen der Teströhrchen.

Durch die Trägervorrichtung des Testkits werden die einzelnen Komponenten des Testkits fixiert. Dies ermöglicht eine übersichtliche Anordnung der einzelnen Komponenten und vereinfacht somit die Durchführung des Tests. Durch die fixierte Anordnung der einzelnen Komponenten in der Trägervorrichtung kann das Testkit als Ganzes aus der Umverpackung herausgeholt werden und ein Herunterfallen der Einzelkomponenten wird vermieden. Weiterhin ist das Testkit durch die Trägervorrichtung sehr übersichtlich gestaltet und ermöglicht eine geordnete Durchführung der Nachweisreaktion auch in stressigen oder unübersichtlichen Einsatzsituationen. Zudem dient die Trägervorrichtung auch als Ablage für die Einzelkomponenten während der Durchführung des Nachweises, so dass der Anwender den Nachweis auch bei geringem Platzangebot einfach durchführen kann.

Die Trägervorrichtung weist hierbei eine Oberseite und eine Unterseite auf. Auf der Oberseite der Trägervorrichtung sind zumindest vier Fixierungsvorrichtungen zum Fixieren der oben beschriebenen Probenröhrchen des Testkits angeordnet. Die Fixierungsvorrichtungen werden durch Aussparungen in der Trägervorrichtung gebildet. Vorzugsweise sind die einzelnen Fixierungsvorrichtungen parallel zueinander und in einer Reihe angeordnet. Die Probenröhrchen werden hierbei parallel zueinander und horizontal zur Oberseite der Trägervorrichtung fixiert. Die Fixierungsvorrichtungen sind so ausgebildet, dass das geschlossene Ende der Probenröhrchen zuerst in die Fixierungsvorrichtungen eingeführt wird. Somit sind die Deckel der Probenröhrchen leicht zugänglich und können abgeschraubt werden, ohne dass das Probenröhrchen aus der Fixierungsvorrichtung entfernt werden muss. Dies ist insbesondere für die Entnahme der Reagenziendeckel mit erster und zweiter reaktiver Komponente vorteilhaft.

Die Positionierung der Probenröhrchen parallel zueinander und horizontal zur Oberseite der Trägervorrichtung ist zudem vorteilhaft, da das Testkit so bei Gebrauch leicht abgelegt werden kann. Zudem kann so ein relativ flaches Testkit mit geringem Platzbedarf erhalten werden. Ein geringer Platzbedarf des Testkits ist hierbei besonders vorteilhaft, wenn das Testkit im Einsatz als Teil der Ausrüstung und/oder am Körper mitgeführt wird. Neben einem geringen Platzbedarf bietet ein flaches Testkit zudem den Vorteil, dass eine Verformung der Umverpackung und der darauf befindlichen Etiketten vermieden und so eine gute Lesbarkeit der Etiketten gewährleistet werden kann. Letzteres ist insbesondere vorteilhaft, da gemäß einer Ausführungsform der Erfindung beispielsweise das Anleitungsschema zur Durchführung des Nachweises, die Farbskala zur Auswertung des Testes und/oder andere relevante Angaben in Form wenigstens eines Etikettes auf der Umverpackung des Testkits aufgebracht sein kann.

Weiterhin weist die Trägervorrichtung auf der Oberseite zumindest zwei Aufnahmevorrichtungen zur Aufnahme der Deckel der Probenröhrchen sowie eine Aufnahmevorrichtung zur vertikalen Aufnahme eines Probenröhrchens auf. Die Aufnahmevorrichtungen sind dabei als Vertiefungen in der Oberseite der Trägervorrichtung ausgebildet. Vorzugsweise weist die Trägervorrichtung eine geschlossene Unterseite auf.

Die Aufnahmevorrichtungen zur Aufnahme der Reagenziendeckel, auch als Deckelhalterung bezeichnet, sind vorzugsweise kreisförmig ausgebildet und weisen einen Durchmesser auf, der größer als der Durchmesser der Reagenziendeckel ist. In den Deckelhalterungen können hierbei die Reagenziendeckel mit den reaktiven Komponenten abgelegt bzw. aufbewahrt werden. Dies bietet neben der besseren Übersichtlichkeit auch die Möglichkeit der Visualisierung der bereits erfolgten Nachweisschritte und verringert somit gerade in stressigen und/oder zeitkritischen Einsatzsituationen die Fehleranfälligkeit des Nachweises durch Anwendungsfehler. Eine Ausführungsform der Erfindung sieht vor, dass die Tiefe der Aussparung h₁ in der Oberfläche, welche die Deckelhalterung bildet, kleiner ist als die Dicke der Trägerhalterung. So wird ein Durchrutschen der Deckel durch die Trägerhalterung vermieden. Alternativ oder zusätzlich ist die Tiefe der Aussparung h₁ kleiner als die Deckelhöhe, so dass nur ein Teil des Deckels von der Deckelhalterung aufgenommen wird. Dies erleichtert ein Entfernen des Reagenziendeckels aus der Deckelhalterung, wenn beispielsweise die Deckelhalterung nur zur temporären Deckelablage dient.

Die Aufnahmevorrichtung zur vertikalen Aufnahme eines Probenröhrchens, auch als Probenhalter bezeichnet, weist die gleiche Querschnittsform wie die Probenröhrchen, insbesondere wie das Probenröhrchen mit der Pufferlösung, auf. Hierbei wird der Durchmesser und Tiefe der Aussparung in der Trägervorrichtung so gewählt, dass das Probenröhrchen in die entsprechende Aufnahmevorrichtung gestellt werden kann. Um eine sichere Aufnahme des Probenröhrchens in die Aufnahmevorrichtung zu gewährleisten, ist es besonders vorteilhaft, wenn die Tiefe h₂ der Vorrichtung größer als die Tiefe h₁ ist.

Vorzugsweise ist die Tiefe der Aussparung jedoch geringer als die Gesamtdicke der Trägervorrichtung. Die Aufnahmevorrichtung zur vertikalen Aufnahme eines Probenröhrchens erfüllt somit die Funktion eines Probenhalters und ermöglicht es, das Probenröhrchen mit der Puffer- bzw. Reaktionslösung zwischen den einzelnen Nachweisschritten sicher in der Trägervorrichtung abzustellen. Somit hat der Anwender für die Durchführung der einzelnen Nachweisschritte beide Hände frei, was die Durchführung des Nachweises erheblich vereinfacht. Zudem wird die Gefahr eines unbeabsichtigten Ausschüttens der Puffer- bzw. Reaktionslösung erheblich verringert.

Es hat sich für die Durchführung des Nachweises als vorteilhaft herausgestellt, wenn die Probenhalterung und die Deckelhalterungen auf der Trägervorrichtung in einer Reihe angeordnet sind. Durch die hierdurch erzielte Übersichtlichkeit können Anwendungsfehler weiter minimiert werden.

Gemäß einer Ausführungsform ist die Trägervorrichtung einstückig ausgebildet. Hierdurch kann die Stabilität der Trägervorrichtung verbessert werden. Eine einstückige Trägervorrichtung kann auch in Hinblick auf den Herstellungsprozess vorteilhaft sein, da beispielsweise auf Fügeprozesse verzichtet werden kann. Vorzugsweise umfasst oder besteht die Trägervorrichtung aus Kunststoffen wie z.B. Acrylnitril-Butadien-Styrol (ABS), Polylactat (PLA), Polypropylen (PP), Polystyrol (PS), Polycarbonat (PC) oder Polyethylen (PE).. Gemäß einer besonders vorteilhaften Ausführungsform ist die Trägervorrichtung ein Spritzgussformteil oder ein 3D-Druck.

Die Fixierungsvorrichtungen zur Aufnahme der Probenröhrchen können insbesondere Klemm- oder Steckvorrichtungen sein. Gemäß einer bevorzugten Ausführungsform sind die Fixierungsvorrichtungen als Klemmvorrichtungen ausgebildet und weisen einen kreisbogenförmigen Querschnitt auf. Eine besonders gute Fixierung des Probenröhrchens in der Fixierungsvorrichtung kann dabei erfolgen, wenn die Bogenlänge des Querschnitts größer als der halbe Umfang des entsprechenden Kreisumfangs ist. Bei dieser Ausführungsform wird das Probenröhrchen stellenweise von der Fixierungsvorrichtung umschlossen. Vorzugsweise sind in dieser Ausführungsform die einzelnen Fixierungsvorrichtungen in einer Reihe angeordnet und zwischen den einzelnen Fixierungsvorrichtungen weist die Trägervorrichtung auf der Oberseite Aussparungen auf, wobei die Aussparungen vorzugsweise die gleiche Länge wie die Fixierungsvorrichtungen aufweisen. Die Aussparungen weisen bevorzugt einen eckigen Querschnitt, besonders bevorzugt einen rechteckigen Querschnitt auf. Auf Grund der Aussparungen zwischen den einzelnen Fixierungsvorrichtungen weisen diese relativ dünne Seitenteile auf. Durch diese Seitenteile erhält die Fixierungsvorrichtung eine gewisse Flexibilität in ihrer Struktur, so dass diese durch die Probenröhrchen stellenweise aufgeweitet werden kann. Somit kann ein Probenröhrchen durch geringe Kraftaufwendung sicher in die Fixierungsvorrichtung eingeklemmt und wieder entfernt werden.

Gemäß einer Ausführungsform enthält das Probenröhrchen mit der Pufferlösung eine Pufferlösung mit einem pH-Wert im Bereich von 6 bis 8, vorzugsweise im Bereich von 7,3 bis 7,5. Insbesondere handelt es sich bei dem Puffer um einen Phosphat-Puffer.

Der Deckel mit der ersten reaktiven Komponente enthält eine Acetylcholinesterase (AChE), vorzugsweise humane Acetylcholinesterase (hAChE). Die Verwendung humaner Acetylcholinesterase hat den Vorteil, dass somit alle für den menschlichen Organismus relevanten Acetylcholinesterasehemmer nachgewiesen werden können. So können nicht nur auf das cholinogene System wirkende Organophosphate, sondern auch beispielsweise Carbamate nachgewiesen werden. Das Testkit ist folglich insbesondere eingerichtet bzw. ausgestattet zum Nachweis eines Nervenkampfstoffes, wie z.B. Sarin, Tabun, VX, Senfgas, Soman, Cyclosarin und/oder Novichok, oder eines Pestizids als Acetylcholinesterasehemmer oder auch anderer die Acetylcholinesterase hemmende Substanz.

### Figurenbeschreibung

Nachfolgend wird die Erfindung anhand der Fig. 1 bis 11 näher beschrieben. Es zeigen:
- Fig. 1: eine schematische Darstellung der Nachweisreaktion von Acetylcholinesterasehemmern mit Hilfe des Testkits,
- Fig. 2: eine schematische Darstellung des Testkits in Aufsicht
- Fig. 3: eine schematische Darstellung der Trägervorrichtung in Aufsicht
- Fig. 4: eine schematische Darstellung eines Querschnitts durch den Teilbereich einer Trägervorrichtung mit Deckelhalter und Probenhalter,
- Fig. 5: eine schematische Darstellung eines Querschnitts durch den Teilbereich einer Trägervorrichtung mit Fixierungsvorrichtungen,
- Fig. 6: eine schematische Darstellung eines Querschnitts durch den Teilbereich einer Trägervorrichtung mit Deckel und Probenröhrchen,
- Fig. 7: eine fotografische Aufnahme einer Umverpackung eines Testkits als Ausführungsbeispiel,
- Fig. 8: eine fotografische Aufnahme eines Testkits als Ausführungsbeispiel,
- Fig. 9: eine fotografische Aufnahme eines Testkits, wobei das Testkit für die Durchführung der Nachweisreaktion vorbereitet wurde,
- Fig. 10: eine fotografische Aufnahme eines umverpackten Testkits aus dem Stand der Technik und
- Fig. 11: eine fotografische Aufnahme der Einzelkomponenten des in Fig. 10 gezeigten Testkits.

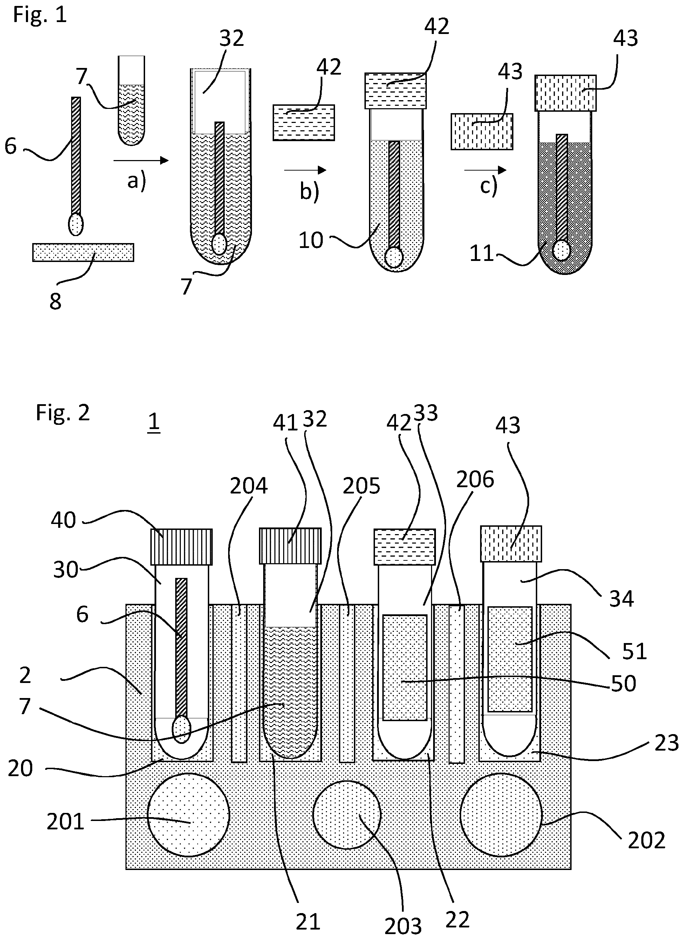

Fig. 1 zeigt eine schematische Darstellung der Durchführung des Tests auf Acetylcholinesterasehemmer, insbesondere eines Nervenkampfstoffes, wie z.B. Sarin, Tabun, VX, Senfgas, Soman, Cyclosarin und/oder Novichok, eines Pestizids oder einer anderen die Acetylcholinesterase hemmende Substanz, mit dem erfindungsgemäßen Testkit. In einem ersten Schritt a) wird die zu untersuchende Oberfläche 8 mit der Probenentnahmevorrichtung 6, beispielsweise einem Wattestäbchen, abgestrichen und die Probenentnahmevorrichtung 6 wird in ein Probenröhrchen 32 mit Pufferlösung 7 gegeben.

Anschließend wird in Schritt b) der Reagenziendeckel 42 auf das Probenröhrchen 32 geschraubt. Im Reagenziendeckel 42 befindet sich als erste reaktive Komponente Acetylcholinesterase.

Nun wird das Probenröhrchen 32 für 10 Sekunden geschüttelt und damit sowohl der eventuell mit der Probenentnahmevorrichtung aufgenommene AChE-Hemmstoff als auch die im Deckel 42 vorhandene Acetylcholinesterase in die Pufferlösung gebracht, so dass sich die Probenlösung 10 bildet.

Nach einer Wartezeit von einer Minute, innerhalb derer der gegebenenfalls vorhandene AChE-Hemmstoff an AChE binden und diese somit ganz oder teilweise hemmen kann, wird im Schritt c) der Reagenziendeckel 42 durch den Reagenziendeckel 43 ersetzt. Im Reagenziendeckel 43 befindet sich als zweite reaktive Komponente DTNB und Acetylthiocholin. Zum Herauslösen der zweiten reaktiven Komponente aus dem Reagenziendeckel 43 wird das Probenröhrchen 32 erneut geschüttelt, so dass die Probenlösung 10 die Komponenten aus dem Reagenziendeckel 43 herauslösen können. Nachfolgend wird die Färbung der so erhaltenen Lösung 11 mit einer Farbtafel verglichen. Eine Gelbfärbung der Lösung 11 wird durch das Ion TNB²⁻ verursacht und zeigt die Anwesenheit einer aktiven, d.h. nicht gehemmten Acetylcholinesterase in der Lösung 11 an. Eine Gelbfärbung ist somit ein Indikator für die Abwesenheit eines Acetylcholinesterasehemmers in Probe 8 und stellt somit einen negativen Test auf Acetylcholinesterasehemmer dar. Enthält die Probe 8 dagegen Acetylcholinesterasehemmer, so bleibt in Schritt c) eine Gelbfärbung der Lösung 11 aus bzw. es kann nur eine sehr schwache Gelbfärbung der Lösung 11 beobachtet werden.

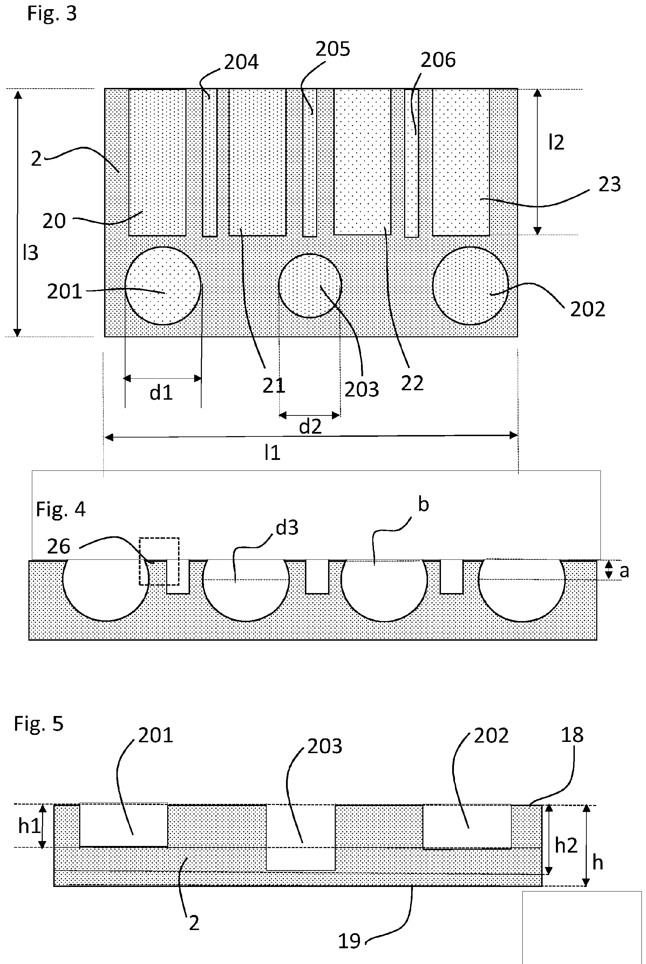

Fig. 2 stellt eine schematische Darstellung des Testkits 1 in Aufsicht dar. Das Testkit 1 umfasst die Trägervorrichtung 2, sowie die Probenröhrchen 30, 32, 33, 34 mit den Deckeln 40, 41 und den Reagenziendeckeln 42, 43. Die Proberöhrchen 30, 32, 33, 34 sind in den Fixierungsvorrichtungen 20, 21, 22, 23 fixiert. Die Fixierungseinrichtungen 20, 21, 22, 23 stellen Aussparungen auf der Oberseite 18 (Fig. 5) der Trägervorrichtung 2 dar und sind parallel zueinander in einer Reihe angeordnet. In dem in Fig. 2 dargestellten Ausführungsbeispiel sind die Fixierungsvorrichtungen 20, 21, 22, 23 als Klemmvorrichtungen ausgebildet. Um die hierfür notwendige Flexibilität der Fixierungsvorrichtungen 20, 21, 22, 23 zum Einklemmen der Probenröhrchen 30, 32, 33, 34 zu gewährleisten, weist die Trägervorrichtung 2 zwischen den Fixierungsvorrichtungen 20, 21, 22, 23 Aussparungen 204, 205, 206 auf der Oberseite 18 der Trägervorrichtung 2 auf.

Die Probenröhrchen 30, 32, 33, 34 werden durch die Fixierungsvorrichtungen 20, 21, 22, 23 parallel zueinander und horizontal zur Oberseite 18 der Trägervorrichtung fixiert. Dabei sind die Probenröhrchen 30, 32, 33, 34 nur teilweise in den Fixierungsvorrichtungen 20, 21, 22, 23 fixiert. Während sich der untere Teil der Probenröhrchen 30, 32, 33, 34 innerhalb der Fixierungsvorrichtungen befindet, ist der obere Teil der Probenröhrchen 30, 32, 33, 34 mit den Deckeln 40, 41 und den Reagenziendeckeln 42, 43 frei zugänglich, so dass zur Entfernung der Deckel 40, 42, 43 nicht zwingend eine Entnahme des Probenröhrchens notwendig ist. Die im Ausführungsbeispiel gezeigte Anordnung der einzelnen Probenröhrchen 30, 32, 33, 34 führt zu einer geringen Gesamtgröße des Testkits.

Die Trägervorrichtung 2 weist zudem die beiden Deckelhalterungen 201, 202 und die Probenhalterung 203 auf, welche als Vertiefungen auf der Oberseite 18 der Trägervorrichtung 2 ausgebildet sind. In dem in Fig. 2 dargestellten Ausführungsbeispiel sind Deckelhalterungen 201, 202 und Probenhalterung 203 in einer Reihe oberhalb der Fixierungsvorrichtungen 20, 21, 22, 23 angeordnet.

Probenröhrchen 30 enthält die Probenaufnahmevorrichtung 6 in Form eines Wattestäbchens, welche durch Öffnen des Deckels 40 leicht entnommen werden kann.

Das Probenröhrchen 32 enthält die Pufferlösung 7. Es ist vorgesehen, dass das Probenröhrchen 32 nach dem Entfernen aus der Fixierungsvorrichtung 21 im Probenhalter 203 platziert wird. Somit hat der Anwender des Testkits 1 bei der Durchführung des Nachweises die Hände frei.

Im Reagenziendeckel 42 befindet sich als erste aktive Komponente Acetylcholinesterase und im Reagenziendeckel 43 als zweite aktive Komponente Ellmans Reagenz sowie Acetylthiocholin. Beide aktive Komponenten liegen in fester Form vor. Die Röhrchen 33, 34 dienen lediglich zur Abschirmung der aktiven Komponenten in den Deckeln 43, 44, so dass diese nicht kontaminiert werden oder herausfallen können. In dem gezeigten Ausführungsbeispiel enthalten die Röhrchen 33, 34 zudem Trockenmittel 50, 51 in Beutelform.

Zur Vorbereitung der Durchführung der Nachweisreaktionen und/oder zur Ablage der leeren Deckel nach dem Herauslösen der aktiven Komponenten dienen die Deckelhalterungen 201, 202.

In dem in Fig. 2 dargestellten Ausführungsbeispiel sind die einzelnen Komponenten bzw. die Probenröhrchen 30, 32, 33, 34 von links nach rechts in der für die Durchführung des Nachweises erforderlichen Reihenfolge gemäß Fig. 1 angeordnet. Dies erleichtert die Durchführung des Nachweises und dient zur Minimierung von Anwenderfehlern.

Fig. 3 zeigt eine schematische Darstellung der bereits in Fig. 2 dargestellten Trägervorrichtung 2. In dem hier dargestellten Ausführungsbeispiel sind die Fixierungsvorrichtungen als Klemmvorrichtung ausgebildet und umfassen die Vertiefungen 20, 21, 22, 23 sowie die Aussparungen 204, 205, 206. Die Vertiefungen 20, 21, 22, 23 dienen zur Aufnahme der Probenröhrchen 30, 32, 33, 34 und weisen ein kreisbogenförmiges Profil auf.

Im dargestellten Ausführungsbeispiel weist die Trägervorrichtung eine Länge I₁ von 80 mm und eine Länge I₃ von 50 mm auf und ist somit sehr kompakt. Der Durchmesser d₁ der Deckelhalterungen 201, 202 beträgt 15 mm und der Durchmesser d₂ weist einen Durchmesser von 12 mm auf. Somit ist die Trägervorrichtung insbesondere für Probenröhrchen mit einem Durchmesser im Bereich von 10 bis 12 mm geeignet.

Fig. 4 zeigt eine schematische Darstellung eines Querschnitts durch die Fixierungsvorrichtung einer wie in Fig. 3 gezeigten Trägervorrichtung 2. Die Sehnenlänge b des Kreisbogens ist hierbei kleiner als der Durchmesser des Kreises d₃ und die Bogenlänge ist größer als der halbe Kreisumfang. Aufgrund dieses Profils kann eine Fixierung von Probenröhrchen mit entsprechenden Durchmessern durch Einklemmen der Probenröhrchen erfolgen. Durch die Aussparungen 204, 205, 206 zwischen den Vertiefungen 20, 21, 22, 23 werden Klemmflügel 26 gebildet, welche die für das Einklemmen des Probenröhrchens benötigte Flexibilität der Fixierungsvorrichtung gewährleistet. Die Länge I₂ der Vertiefungen 20, 21, 22, 23 ist vorzugsweise geringer als die Länge der Probenröhrchen 30, 32, 33, 34. Der Durchmesser der Probenröhrchen entspricht dabei dem Durchmesser d₃ oder ist geringfügig kleiner.

Fig. 5 zeigt eine schematische Darstellung eines Querschnitts durch einen Teilbereich einer wie in Fig. 3 gezeigten Trägervorrichtung 2. Der in Fig. 5 gezeigte Teilbereich umfasst hierbei die Deckelhalterungen 201 und 202 sowie die Probenhalterung 203. Sowohl Deckelhalterungen 201, 202 als auch die Probenhalterung 203 werden durch Aussparungen in der Oberseite 18 der Trägervorrichtung 2 gebildet. In dem in Fig. 5 dargestellten Ausführungsbeispiel weisen die Deckelhalterungen 201, 202 eine Tiefe h₁ und die Probenhalterung 203 eine Tiefe h2 auf, wobei die Tiefe h₂ größer ist als die Tiefe h₁. Die Deckelhalterungen 201, 202 sind somit flacher ausgebildet als die Probenhalterung 203. Mit anderen Worten ist die Probenhalterung 203 bevorzugt mit einer größeren Tiefe ausgebildet als die Deckelhalterungen 201, 202, insbesondere auch, um eine sichere Aufnahme des Probenröhrchens in die Aufnahmevorrichtung zu gewährleisten. Sowohl Deckelhalterungen 201, 202 als auch Probenhalterung 203 weisen eine Tiefe auf, die geringer als die Dicke h der Trägervorrichtung ist. Somit weist das in den Fig. 3 bis 5 dargestellte Ausführungsbeispiel eine geschlossene Unterseite auf. Dies ist vorteilhaft, da somit sowohl Deckel als auch Probenröhrchen unabhängig vom jeweiligen Untergrund, auf den die Trägervorrichtung 2 gelegt wird, fixiert werden.

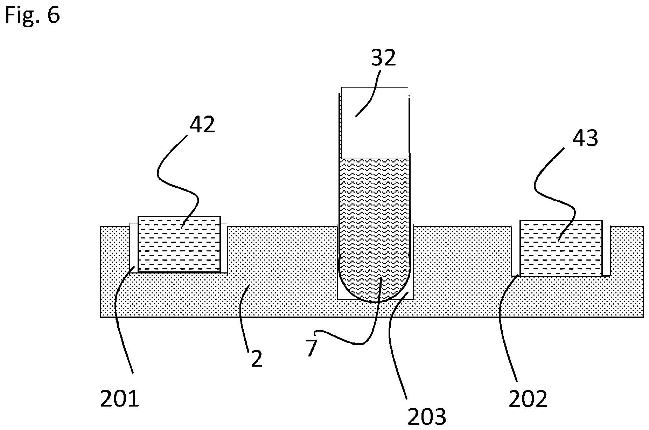

Fig. 6 zeigt eine schematische Darstellung des in Fig. 5 gezeigten Ausschnitts, bei der die Deckel 42, 43 in den Deckelhalterungen 201 und 202 positioniert wurden. In der Probenhalterung 203 steckt das Probenröhrchen 30 mit der Pufferlösung 7.

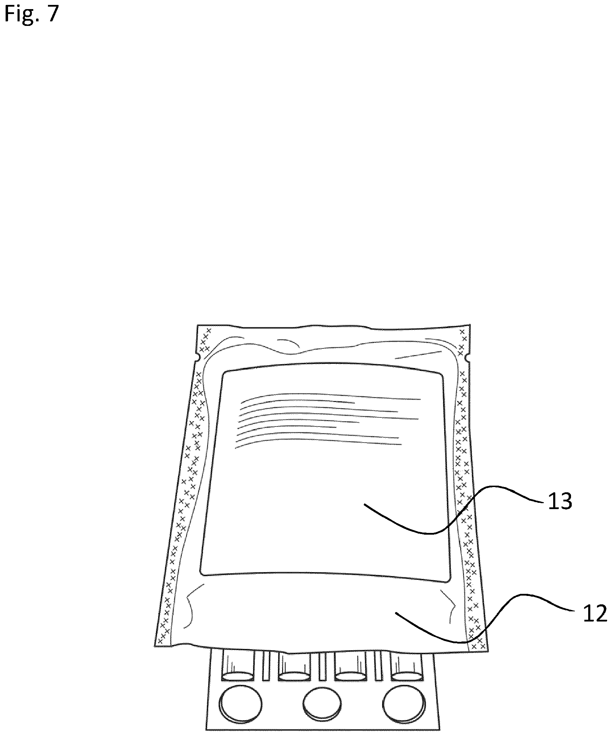

Fig. 7 zeigt eine fotografische Aufnahme eines Ausführungsbeispiels, bei dem das Testkit 1 in einer Folienumverpackung 12 verpackt ist. Durch Verwendung der erfindungsgemäßen Trägervorrichtung 2 kann das Testkit 1 flach verpackt werden. Auf der Umverpackung 12 befindet sich zweckmäßig auf einer Seite (Vorder- oder Rückseite) ein Etikett 13, welches eine Kurzbeschreibung und ein Anleitungsschema zur Durchführung des Tests enthält. Auf der anderen Seite der Umverpackung 12 (bei Fig. 7 folglich nicht zu sehen) befindet sich zweckmäßig ein Etikett mit den relevanten Angaben (z.B. Bezeichnung, Herstellung, Chargen-Nr., Haltbarkeit) und/oder der Farbskala. Dadurch, dass das Testkit flach ist, sind die Etiketten bereits vor dem Entfernen des Testkits aus der Umverpackung gut lesbar.

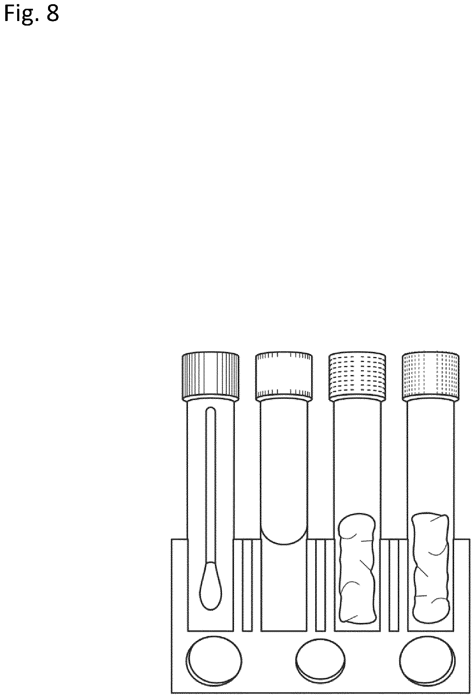

Fig. 8 ist eine fotografische Aufnahme, die den Zustand des Testkits 1 nach Entnahme aus der Umverpackung zeigt. Durch die Fixierung der einzelnen Probenröhrchen können nicht nur die Abmaße des Testkits minimiert werden, sondern die einzelnen Komponenten des Testkits werden dem Anwender darüber hinaus in einer sehr übersichtlichen Art präsentiert. Durch die Fixierung der einzelnen Komponenten durch die Trägervorrichtung wird zudem ein Vertauschen der Komponenten vermieden. In dem in Fig. 8 gezeigten Ausführungsbeispiel sind die Einzelkomponenten wiederum entsprechend der Fig. 2 von links nach rechts in der für den Test benötigten Reihenfolge angeordnet. Dies vereinfacht ebenfalls die Anwendung und minimiert Anwenderfehler.

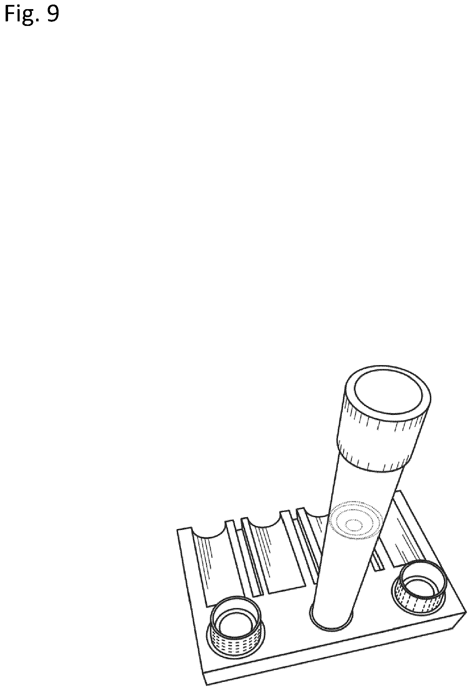

Fig. 9 zeigt eine fotografische Aufnahme des in Fig. 8 gezeigten Ausführungsbeispiel, bei dem die einzelnen Komponenten in Vorbereitung auf die Testdurchführung in der Trägervorrichtung angeordnet wurden. Die Reagenziendeckel mit den aktiven Komponenten Acetylcholinesterase, DTNB und Acetylthiocholin wurden dabei in den Deckelhaltern der Trägervorrichtung platziert. Das Probenröhrchen wurde in die Probenhalterung gesteckt. Der Anwender kann somit die Probe mit der Probenentnahmevorrichtung entnehmen und sich auf die Durchführung der Nachweisreaktion konzentrieren, ohne zwischen den einzelnen Schritten die Einzelkomponenten herauszusuchen und/oder auszupacken. Da die Trägervorrichtung eine sichere Ablage für Probenröhrchen und die Deckel mit den aktiven Komponenten bildet, kann eine Kontamination oder ein Verschütten, insbesondere bei unruhigen Einsatzumgebungen vermieden werden.

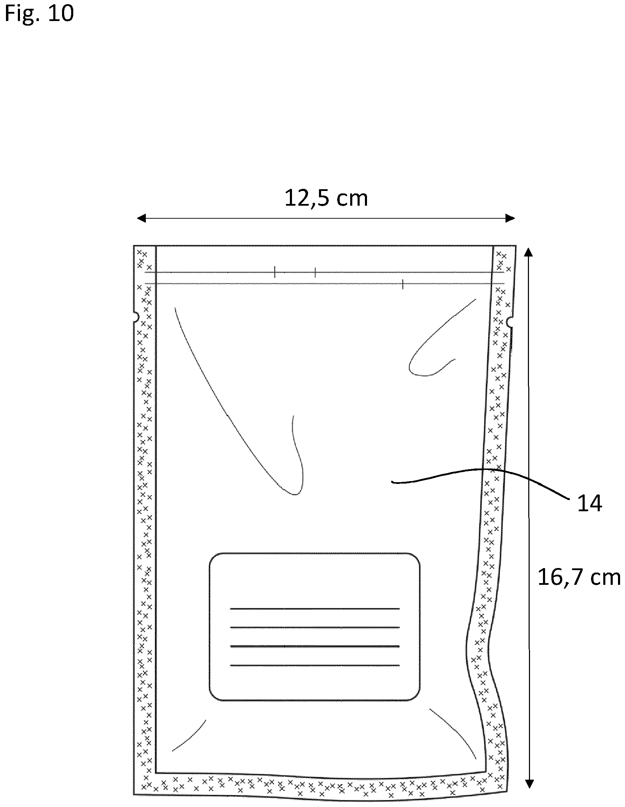

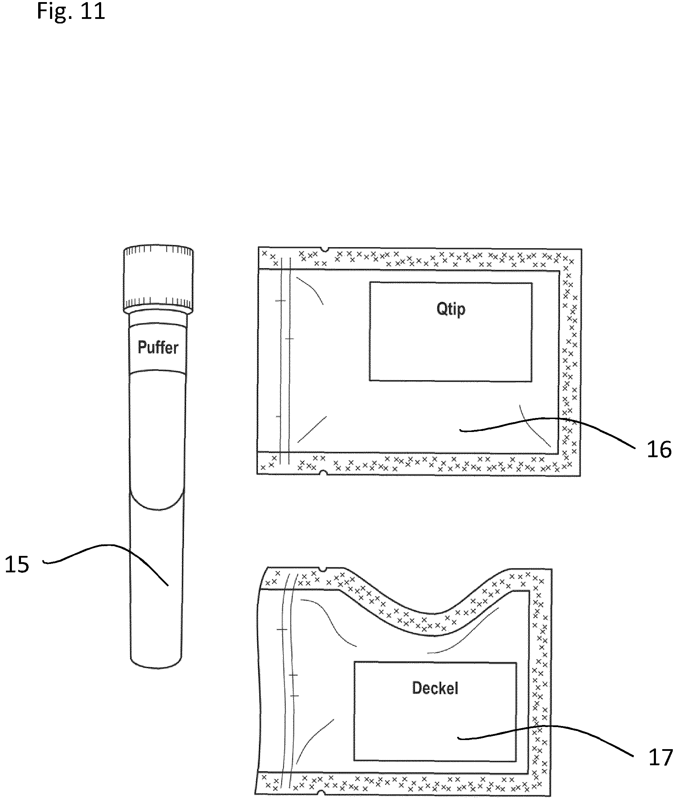

In den Fig. 10 und 11 werden fotografische Aufnahmen eines bekannten Testkits als Vergleich gezeigt. Fig. 10 zeigt das Testkit in einer Folienumverpackung 14. Durch die lose Anordnung der Einzelkomponenten weist die Umverpackung 14 eine deutliche Wölbung auf. Fig. 11 zeigt die Einzelkomponenten, bestehend aus Probenröhrchen mit Pufferlösung 15, eingeschweißter Probenentnahmevorrichtung 16 und folienverschweißter Umverpackung 17, welche die Reagenziendeckel mit den aktiven Komponenten Acetylcholinesterase, DTNB und Acetylthiocholin enthält.

### Bezugszeichenliste

- 1: Testkit
- 2: Träger
- 6: Probenentnahmevorrichtung
- 7: Pufferlösung
- 8: Probe
- 10: Probenlösung mit Acetylcholinesterase
- 11: Testlösung mit Probenlösung 10, 5,5'-Dithiobis-2-nitrobenzoesäure und Acetylthiocholin
- 12: Umverpackung
- 13: Etikett
- 14: Umverpackung Vergleichsbeispiel
- 15: Testlösung des Vergleichsbeispiels
- 16: eingeschweißte Probenentnahmevorrichtung des Vergleichsbeispiels
- 17: eingeschweißte Deckel des Vergleichsbeispiels
- 18: Oberseite der Trägervorrichtung
- 19: Unterseite der Trägervorrichtung
- 20, 21, 22, 23: Fixierungsvorrichtung
- 30, 32, 33, 34: Probenröhrchen
- 40, 41: Deckel
- 42: Reagenziendeckel mit Acetylcholinesterase
- 43: Reagenziendeckel mit 5,5'-Dithiobis-2-nitrobenzoesäure und Acetylthiocholin
- 50, 51: Trockenmittel
- 201, 202: Aufnahmevorrichtung für Reagenziendeckel
- 203: Probenhalter
- 204, 205, 206: Aussparung

## Patentansprüche

1. Testkit zur Detektion von Organophosphatverbindungen umfassend eine Trägervorrichtung (2) mit einer Oberseite (18) und einer Unterseite (19), eine Probenentnahmevorrichtung (6), eine Pufferlösung (7) in einem Probenröhrchen (32) mit Deckel (41), sowie einer Acetylcholinesterase als erste aktive Komponente und einer Mischung aus 5,5'-Dithiobis-2-nitrobenzoesäure (DTNB) und Acetylthiocholin, wobei die Probenentnahmevorrichtung (6) in einem Probenröhrchen (30) mit Deckel (40) verpackt ist, die erste aktive Komponente in einem ersten Reagenziendeckel (42) und die zweite aktive Komponente in einem zweiten Reagenziendeckel (43) appliziert ist und der erste und der zweite Reagenziendeckel (42, 43) jeweils ein Probenröhrchen (33, 34) verschließt, wobei die Trägervorrichtung zumindest vier Fixierungsvorrichtungen (20, 21, 22, 23) zur Aufnahme der Probenröhrchen (30, 32, 33, 34) aufweisen, wobei die Fixierungsvorrichtungen(20, 21, 22, 23) durch Aussparungen in der Trägervorrichtung (2) gebildet werden, die Probenröhrchen parallel zur Oberseite der Trägervorrichtung (2) fixiert werden und wobei die Trägervorrichtung zumindest zwei Aufnahmevorrichtungen (201, 202) zur Aufnahme des ersten und des zweiten Reagenziendeckels (42, 43) und eine Aufnahmevorrichtung (203) zur vertikalen Aufnahme eines Probenröhrchens (30) aufweisen.

2. Testkit gemäß dem vorherigen Anspruch, wobei die Trägervorrichtung (2) Kunststoff, bevorzugt ein Kunststoff wie z.B. Acrylnitril-Butadien-Styrol (ABS), Polylactat (PLA), Polypropylen (PP), Polystyrol (PS), Polycarbonat (PC) oder Polyethylen (PE), enthält.

3. Testkit gemäß einem der vorstehenden Ansprüche, wobei die Trägervorrichtung (2) einstückig ausgebildet ist.

4. Testkit gemäß einem der vorstehenden Ansprüche, wobei die Fixierungsvorrichtungen (20, 21, 22, 23) zur Aufnahme der Probenröhrchen (30, 32, 33, 34) als Vertiefungen in der Oberseite der Trägervorrichtung (2) ausgebildet sind und die einzelnen Fixierungsvorrichtungen (20, 21, 22, 23) bevorzugt in einer Reihe auf der Trägervorrichtung angeordnet sind.

5. Testkit gemäß einem der vorstehenden Ansprüche, wobei die Fixierungsvorrichtungen (20, 21, 22, 23) als Klemm- oder Steckvorrichtungen ausgebildet sind.

6. Testkit gemäß dem vorstehenden Anspruch, wobei die Fixierungsvorrichtungen (20, 21, 22, 23) als Klemmvorrichtungen ausgebildet sind, einen kreisbogenförmigen Querschnitt aufweisen und bevorzugt die Bogenlänge des Querschnitts größer als der halbe Umfang des entsprechenden Kreises ist.

7. Testkit gemäß einem der vorstehenden Ansprüche 5 bis 6, wobei die Trägervorrichtung (2) zwischen den einzelnen Klemmvorrichtungen (20, 21, 22, 23) Aussparungen (204, 205, 206) auf der Oberseite aufweist und die Aussparungen bevorzugt einen eckigen oder runden, besonders bevorzugt einen rechteckigen Querschnitt aufweisen.

8. Testkit gemäß einem der vorstehenden Ansprüche, wobei die Aufnahmevorrichtungen zur Aufnahme der Reagenziendeckel (42, 43) als Vertiefungen auf der Oberseite der Trägervorrichtung (2) ausgebildet sind und bevorzugt kreisförmig ausgebildet sind, wobei die Kreisdurchmesser größer als der Durchmesser der Reagenziendeckel (42, 43) ist.

9. Testkit gemäß dem vorstehenden Anspruch, wobei die Tiefe h₁ der Vertiefung kleiner als die Höhe der Reagenziendeckel (42, 43) ist.

10. Testkit gemäß einem der vorstehenden Ansprüche, wobei die Vorrichtung zur Aufnahme des Teströhrchens (203) als kreisförmige Vertiefung auf der Oberseite der Trägervorrichtung (2) ausgebildet ist.

11. Testkit gemäß einem der vorstehenden Ansprüche, wobei die Vorrichtung zur Aufnahme des Teströhrchens (203) und die Vorrichtungen zur Aufnahme der Reagenziendeckel (201, 202) in einer Reihe angeordnet sind,
und/oder,
wobei die Trägervorrichtung (2) eine geschlossene Unterseite aufweist,
und/oder,
wobei Pufferlösung einen pH-Bereich von 6 bis 8 aufweist,
und/oder,
wobei die Acetylcholinesterase humane Acetylcholinesterase ist.

12. Testkit gemäß einem der vorstehenden Ansprüche, wobei das Testkit (1) in einem Folienbeutel als Verpackung (12) eingeschweißt ist und/oder auf der Verpackung (12) wenigstens ein Etikett (13) aufgebracht ist.

13. Testkit gemäß einem der vorstehenden Ansprüche, wobei das verpackte Testkit flach verpackt ist,
und/oder,
wobei die Probenröhrchen (33, 34), welche die mit den Deckeln (42, 43) verschlossen werden, ein Trockenmittel (50), vorzugsweise ein umverpacktes Trockenmittel, enthalten, und/oder;
wobei die Probenröhrchen (30, 32, 33, 34) ein Schraubgewinde aufweisen,
und/oder,
wobei die Deckel (40, 41, 42, 43) als Schraubdeckel ausgebildet sind.

14. Testkit gemäß einem der vorstehenden Ansprüche, eingerichtet zum Nachweis eines Acetylcholinesterasehemmers, insbesondere eines Nervenkampfstoffes, wie z.B. Sarin, Tabun, VX, Senfgas, Soman, Cyclosarin und/oder Novichok, eines Pestizids oder einer anderen die Acetylcholinesterase hemmende Substanz sein kann.

15. Verfahren zum Nachweis eines Acetylcholinesterasehemmers mit einem Testkit gemäß einem der vorstehenden Ansprüche umfassend zumindest die folgenden Schritte a) bis c):
a) Abstreifen der zu untersuchenden Oberfläche mit einer Probenentnahmevorrichtung (6),
b) Platzieren der Probenentnahmevorrichtung aus Schritt a) in einem Probenröhrchen (32), wobei das Probenröhrchen (32) eine Pufferlösung (7) enthält,
c) verschließen des Probenröhrchens (32) mit einem ersten Reagenziendeckel (42) und schütteln des mit dem Reagenziendeckels (42) verschlossenen Probenröhrchens (32), wobei sich im Reagenziendeckel (32) als erste reaktive Komponente Acetylcholinesterase befindet und durch das Schütteln ggfs. vorhandener AChE-Hemmstoff aus der Probenentnahmevorrichtung (6) und die im Reagenziendeckel (42) enthaltene Acetylcholinesterase in die Pufferlösung (7) gebracht wird, so dass sich eine Probenlösung (10) bildet,
d) stehenlassen des Probenröhrchens (32) um eine Bindung des eventuell in der Probenlösung (10) enthaltenen Ache-Hemmstoffs an die Acetylcholinesterase,
e) ersetzen des Reagenziendeckels (42) durch einen zweiten Reagenziendeckels (43), wobei der zweite Reagenziendeckel (43) als zweite Komponente DTNB und Acetylthiocholin enthält, und verschließen des Probenröhrchens (32) mit dem zweiten Reagenziendeckel (43),
f) schütteln des mit dem zweiten Reagenziendeckel (43) verschlossenen Probenröhrchens (32) zum Herauslösen der zweiten reaktiven Komponente aus dem zweiten Reagenziendeckels (43), so dass eine Lösung (11) erhalten wird,
g) visueller Vergleich der Färbung der in Schritt f) erhaltenen Lösung (11) mit einer Farbtafel, wobei eine farblose Lösung (11) die Anwesenheit eines Acetylcholinesterasehemmers detektiert
